# EUROPEAN PATENT SPECIFICATION

(11) **EP 0 740 537 B1**
(45) Date of publication and mention of the grant of the patent: **02.07.2003**
(21) Application number: 95903519.7
(22) Date of filing: 01.12.1994
(51) Int. Cl.: A61F 2/00

(54) **NONSURGICAL INTRAURETHRAL BLADDER CONTROL DEVICE**
NICHTCHIRURGISCHE INTRAURETHRALE BLASENKONTROLLVORRICHTUNG
DISPOSITIF INTRA-URETRAL NON CHIRURGICAL DE CONTROLE DE LA VESSIE

(30) Priority: 23.12.1993 US 173636; 30.08.1994 US 298033
(43) Date of publication of application: 06.11.1996
(73) Proprietor: H.K. MEDICAL TECHNOLOGIES INCORPORATED, San Antonio, TX 78217 (US)
(72) Inventor: KULISZ, Andre, A., San Antonio, TX 78248 (US)
(74) Representative: Manresa Val, Manuel
(86) International application number: US9412822
(87) International publication number: WO95017143

(56) References cited:
- US-A- 3 731 670
- US-A- 4 553 533
- US-A- 4 679 546
- US-A- 4 781 176
- US-A- 4 968 294
- US-A- 5 140 999
- US-A- 5 236 446

## Description

### BACKGROUND OF THE INVENTION

**Field of the Intention** - The present invention generally relates to the field of medical devices and more particularly to bladder control devices, and still more particularly to an intraurethral bladder control apparatus commonly referred to as an artificial sphincter.

**Description of the Prior Art -** The use of various sphincter and bladder control devices is wide spread in the field of the present invention. The use of intraurethral valving apparatus is also well known in the art, as evidenced by, for example, U.S. Patent Nos. 4,553,533; 4,969,474; and 5,123,428. In general terms, it is the goal of the prior art and the present invention to provide a valving system for a patient's bladder, which system is placed directly in the urethra and adjacent the bladder. The valving system is ideally turned on by the patient and turned off when the bladder has been sufficiently emptied.

Some of the problems and disadvantages found in the prior art include: the need for surgical implantation and removal of the device in the urethra; the susceptibility of the device to leakage or to undesired valve openings; the failure of the valve device to stay open long enough to provide complete emptying of the bladder; and in some prior art devices the need for an additional external product, such as a magnet, to actuate the valve device.

An apparatus according to the preamble of claim 1 is taught by U.S. Patent No. 4,679,546.

### SUMMARY OF THE INVENTION

The apparatus of this invention overcomes these potential disadvantages by providing an improved intraurethral bladder control apparatus which includes a valve assembly, and a valve assembly mount for releasibly holding the assembly and adapted to be nonsurgically placed and releasibly held in the urethra of a patient. The mount is provided with a textured outer surface to which urethral tissue will conform to hold the mount at a selected position therein. The valve assembly cooperates with apparatus in an inner chamber of the mount for releasable installation of the assembly. The mount is preferably generally cylindrical in shape with a generally cylindrical inner chamber or lumen where valve assembly holding apparatus is deployed.

The valve assembly has adjustment apparatus operable whether the assembly is in the valve assembly mount or not, for positioning the assembly and for setting desired values of the opening and closing the valve, and for a fail-safe mode.

The present invention also includes apparatus for assuring that once opened by the patient's own abdominal muscle contraction, the valve stays open for a sufficient time to empty the bladder, and then closes without further action by the patient. The invention features a design to utilize Bernoulli's principle to provide a negative pressure that holds the valve open during fluid flow, and a second preferred embodiment is designed to utilize a valve area enlargement during fluid flow to keep the valve open.

Additional apparatus for sizing the mount, and for inserting and removing the mount is incorporated into this invention. Pressure apparatus for providing either a liquid, jell or a gas lubricant under pressure is part of this additional apparatus. A number of probes of various sizes, each of which have a lumen extending from the proximal end to sets of aligned holes spaced about the circumference near the distal end, provide a passage for the lubricant. The pressure apparatus is attached to the proximal end of a probe. This provides lubricant about the distal end of the probe. This lubricant aids in inserting probes into the urethral opening in sequence, from the smallest size to the largest size the urethra will accept, to determine the urethra size. This information is necessary to determine the proper mount size.

This same pressure apparatus is used to provide pressurized lubricant adjacent to the base of the mount for placing the mount within the urethra or for removing the mount from the urethra. A retriever, which threads into a mating hole in the base of the mount, has a shoulder at its distal end adjacent to the mount. The retriever has a lumen from the proximal end which communicates with holes spaced about the circumference of the shoulder. The retriever has means at the proximal end for connecting the pressure apparatus. With the pressure apparatus connected to the proximal end of the retriever and the retriever threaded into the base of the mount, the retriver can introduce lubricant under pressure at the base of the mount.

A sleeve with flanges near each end slideably encircles the retriever. This sleeve, when placed over the retriever and pressed against the patient's body, will seal the pressurized lubricant within the urethra. This pressurized lubricant will expand the urethra from the base of the mount inward. This will free the urethra from the textured outer surface of the mount for insertion or removal. The pressurized lubricant will also flow from the base of the mount into this expanded area around the mount to provide lubricant for insertion or removal of the mount. This process is particularly critical when removing the mount, since the walls of the urethra adhere to and tightly engage the textured walls of the mount after installation.

### BRIEF DESCRIPTION OF THE DRAWINGS

The above described as well as other objects and many of the attendant advantages of the present invention will be readily appreciated as they become better understood by reference to the following detailed description when considered in connection with the accompanying drawings, in which like reference numerals designate like parts throughout all figures:
**Fig. 1** is a partial sectional view of a first embodiment of the intraurethral bladder control apparatus of this invention with the valve closed;
**Fig. 2** is a cross-sectional view of **Fig. 1** taken along the line 2;
**Fig. 3** is another cross-sectional view of **Fig. 1** taken along the line 3;
**Fig. 4** is yet another cross-sectional view of **Fig. 1** taken along the line 4;
**Fig. 5** is a partial sectional view of the embodiment of **Fig. 1** with the valve open;

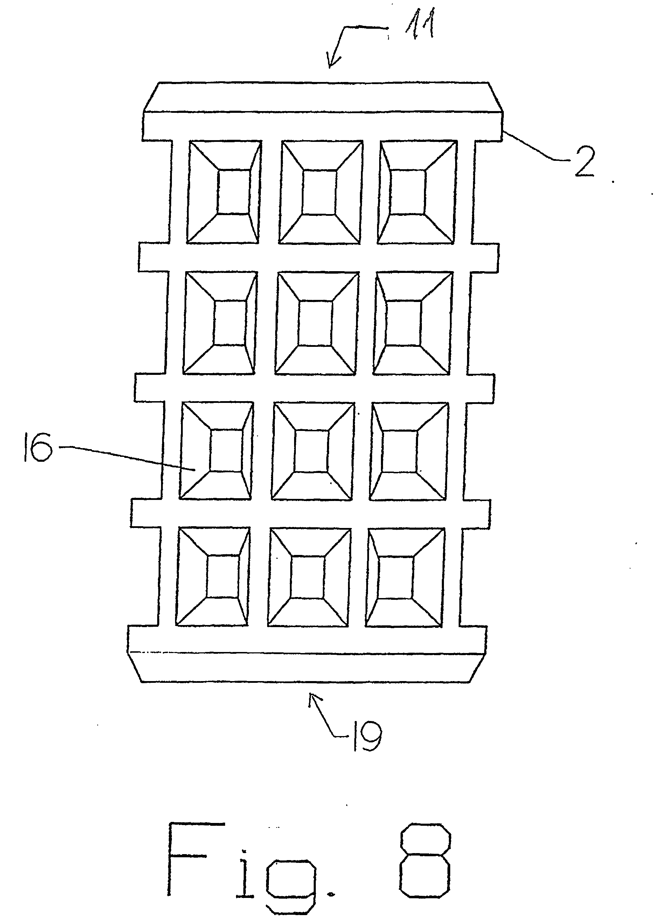

**Fig. 6 and 7** have been left out;
**Fig. 8** is a plan view of the valve assembly mount of this invention;
**Fig. 9** is a plan view of a valve assembly of this invention;
**Fig. 10** is a plan view of pressure apparatus of this invention;
**Fig. 11** is a side view of a retriever;
**Fig. 11A** is an end view of the retriever's distal end.
**Fig. 12** is a side view of a probe;
**Fig. 12A** is a cross-sectional view of **Fig. 12** taken along the line A-A.
**Fig. 13** is a fragmentary view of a patient showing the bladder and urethra in cross-section, with a mount, retriever and sleeve in place within the urethra, and with the proximal end of the retriever connected to the distal end of the distal hose of the pressure apparatus;
**Fig. 14** is a fragmentary view of a patient showing the bladder and urethra in cross-section, with a probe in place within the urerthra, and with the proximal end of the probe connected to the distal end of the distal hose of the pressure apparatus;
**Fig. 15** is a side view of the sleeve;
**Fig. 15A** is the end view of the sleeve's distal end, and
**Fig. 16** is a pressure versus time diagram for three different probes.

### DETAILED DESCRIPTION OF THE PREFERRED EMBODIMENTS

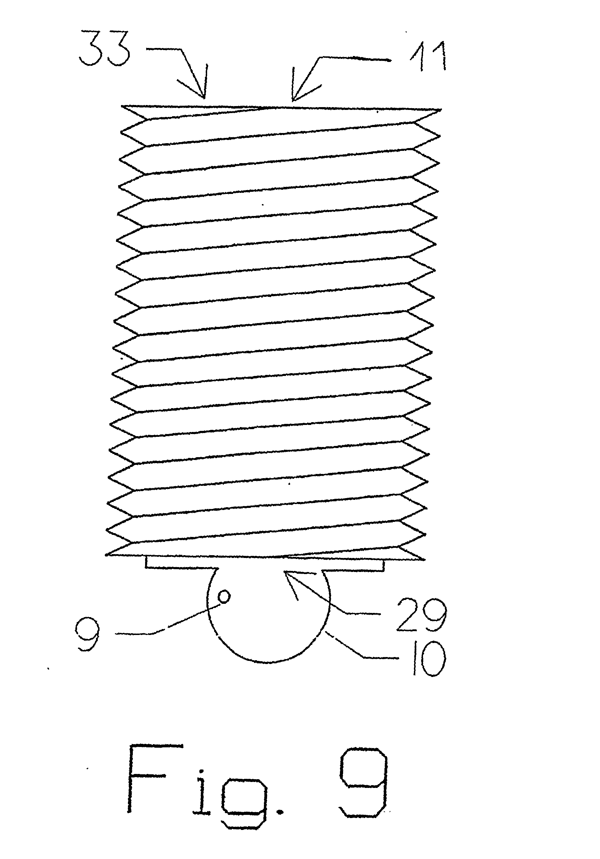

Referring first to **FIG. 8** there is shown a valve assembly mount 2 in the preferred form of a cylinder having a textured outer surface 16 designed to interact with urethral tissues such that mount 2 may be nonsurgically and releasibly positioned in a patient's urethra. Mount 2 includes a valve orifice indicated at 11 and adapted to be positioned adjacent the patient's bladder exit, and an assembly orifice indicated at 19 adapted to receive a valve assembly for internal mounting. As disclosed and more fully described in other figures of the drawings, the interior of mount 2 includes connection apparatus for releasibly and adjustably mounting a valve assembly. In practice, mount 2 is positioned in the patient's urethra first, left for a period of time to enable the urethra tissue to conform to the textured outer surface 16 of mount 2 to hold mount 2 securely in place, and then a valve assembly is mounted in mount 2.

In **Fig. 9** there is shown a valve block 33 adapted to releasibly receive a valve apparatus in a manner fully described below with reference to other figures of the drawings. Block 33 is shown as preferably having a threaded outer surface designed to match a threaded inner surface (not shown in **Fig.8**) of mount 2. Block 33 also has a valve orifice indicated at 21 for alignment adjacent to orifice 11 of mount 2, and an apparatus receiving orifice indicated at 29 for insertion of a valve apparatus.

As can be seen in **Fig. 9**, when a valve apparatus is in place within block 33, an adjustment device 10 having lock notches 9 will be available outside block 33. By using an appropriate adjustment tool (not shown) to lock into notches 9, device 10 may be rotated to make a plurality of adjustments more fully described below with reference to other figures of the drawings.

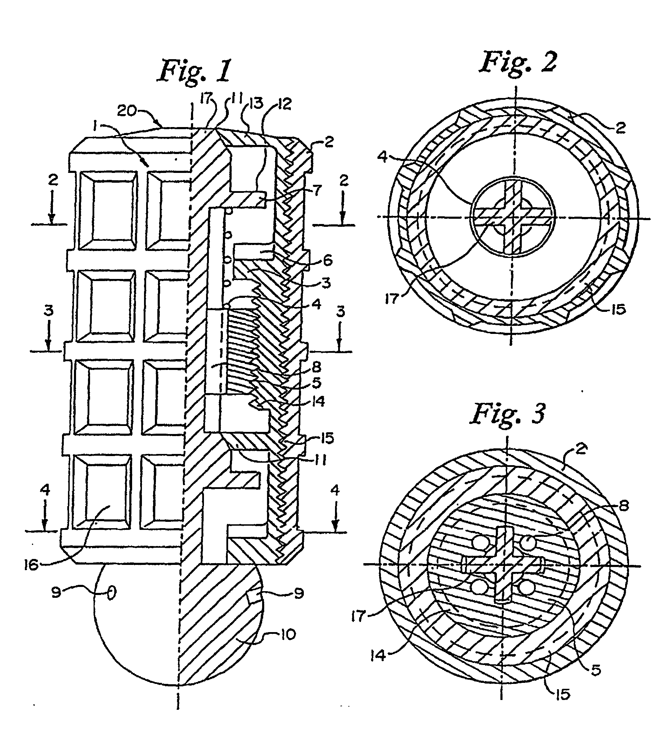

Referring now to **Fig. 1** there is shown a first embodiment of the bladder control apparatus 1 of this invention, including cylindrical valve assembly mount 2 having textured outer surface 16 and a valve assembly 20 which is threaded into mount 2 by means of a connection thread 15. Valve assembly 20 includes a valve block 13, a valve 17, a valve orifice 11, a valve ring 7 having a valve area 12, a coiled spring or other biasing apparatus 4, a spacer 6, a stationary ring 3, a fluid passage 8, a bias adjustment apparatus 5, a thread 14 joining bias adjustment apparatus 5 to block 13, and adjustment device 10 having locking notches 9.

The cross-sectional views of **Figs. 2, 3 and 4**, respectively taken along the lines 2, 3 and 4, more clearly depict the inner structure of device 1 of **Fig. 1**, clarifying the positioning and formations of valve 17, spring coil biasing device 4, bias adjustment means 5 with thread 14, fluid passages 8, stationary ring 3 and spacers 6.

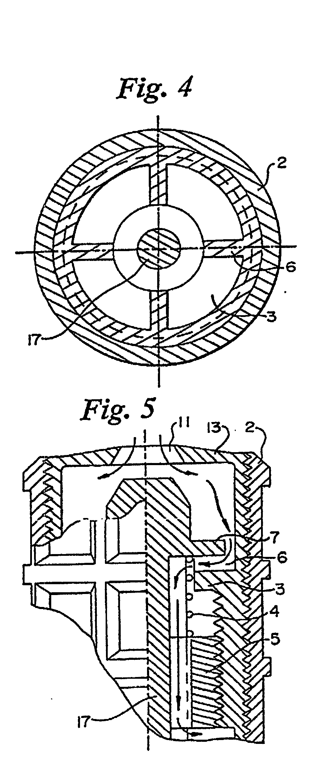

In **Fig. 1** valve 17 is in the closed position in orifice 11, thus preventing the flow of urine fluids from the bladder. With reference to **Fig. 5**, valve 17 is shown in the open position, removed from orifice 11, and a plurality of lines with arrows depict the fluid flow path through valve block 13.

In practice, valve assembly mount 2 is first inserted into a urethra and allowed to stay for a period of time (usually a few weeks) sufficient for the urethral tissue to conform to the textured outer surface 16 to thus essentially immobilize mount 2. After mount 2 is firmly held by the tissue, a valve assembly such as assembly 20 of **Fig. 1** is inserted into the internal chamber or lumen of mount 2 where it is connected and adjusted.

Referring now to **Figs. 1-5**, it is shows how to utilize Bernoulli's principle to retain the valve in an open position, assume that assembly 20 has been mounted in the lumen of assembly mount 2 which has been immobilized in the patient's urethra in the manner described above. Block 13 is then adjusted within mount 2 such that orifice 11 passes through orifice 21, as shown in **Fig. 8,** to align with the output from the patient's bladder (not shown). As the bladder fills with urine, the resulting growth in the urine column exerts greater pressure on valve 17 through orifice 11 and at the same time the resulting growth in volume within the bladder stimulates the patient's need to void. To initiate the voiding process the patient need only contract the muscles of the lower abdominal cavity for a short period of a few seconds. This short period of contraction will increase the pressure on valve 17 long enough for it to move against the bias of spring 4 and thus begin the flow of urine through valve assembly 20 along the path shown by the allows in **Fig. 5**.

As the urine flows in the path shown, valve 17 moves toward valve stop or rest 6. Rest 6 is a vertical element extending radially for separating the lower surface of valve ring 7 from stationary ring 3. The urine then flows around the edge of valve ring 7 and between the lower surface of ring 7 and stationary ring 3, such that a negative pressure is induced according to Bernoulli's principle between these two surfaces. This negative force overcomes the tension of spring 4 and holds ring 7 down against stop 6, thus holding valve 17 open, as long as a sufficient flow of urine is present. When the flow decreases below a sufficient amount, the negative force is decreased until the bias from spring 4 can again close valve 17 in orifice 11 to cut off the flow entirely.

The result of the actions as described in the preceding paragraph causes the desired holding pattern for the artificial sphincter device of this invention. That is, once valve assembly 20 has been opened by muscle contraction to allow fluid flow through block 13, the application of Bernoulli's law will automatically prevent closure of valve 17 without further muscle contraction until the bladder has emptied enough to significantly reduce the urine flow; and then the valve assembly will automatically close without further muscle effort. The patient's need to void will have been met by a simple, short initial contraction of the muscles of the lower abdominal cavity.

The force with which valve 17 is held closed or seated in orifice 11 is determined by the tension from spring 4, which may be any form of bias device. This tension or bias is adjusted by bias adjustment apparatus 5, best seen in Figs. 1 and 3. To set the desired tension of coil 4, an adjustment tool (not shown) is passed through the urethra to grasp and lock into notches 9 of adjustment device 10. Device 10 is then selectively rotated which rotates valve 17 and bias adjustment means 5. The rotation of means 5 causes it to move up or down thread 14 to increase or decrease the tension of spring 4.

The rotation of valve 17 in the direction of reduced bias from coil 4 will eventually disable bladder control device 1 by removing valve 17 from is seat in orifice 11 to allow a free flow of fluid through block 13. Further rotation after reaching the point of free flow will cause rotation of block 13 to first change its positioning within mount 2 and eventually to cause it to disengage from mount 2 entirely. Replacement of block 13 into mount 2 is accomplished by simply reversing the direction of rotation of device 10.

The above description of the structure and operation of the inventive apparatus shown in **Figs. 1-3** which utilizes Bernoulli's law is applicable to patients who have a sufficiently rapid rate and volume of urine flow. These values can be clinically determined.

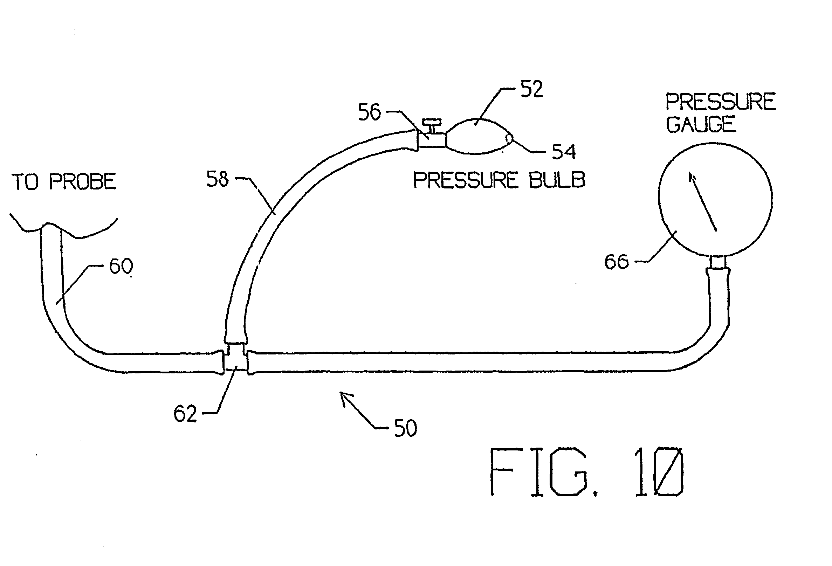

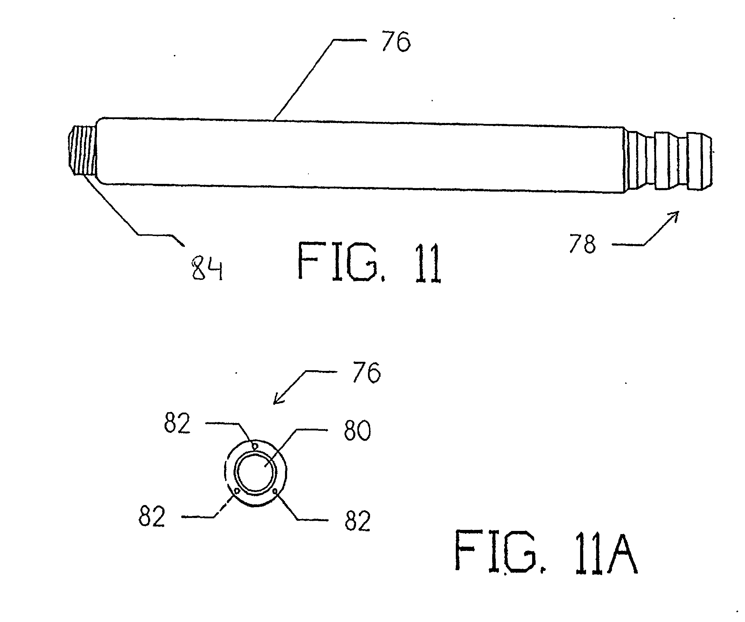

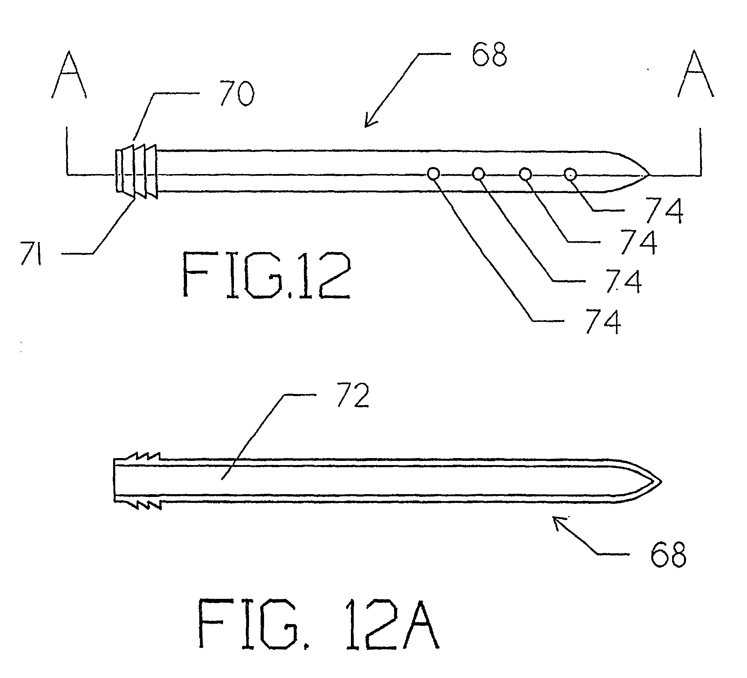

The additional apparatus for sizing the mount, and for inserting and removing the mount is shown in **Figs. 10** through **15A**. Pressure apparatus 50 is shown in **Fig. 10**. Pressure apparatus 50 is used to provide a pressurized liquid, jell or a gas. Pressure apparatus 50 consists of a pressure bulb 52 having an gas intake opening 54 and a shut-off valve 56. A flexible proximal hose 58 and a flexible distal hose 60 both connect to T-connector 62. T-connector 62 is also connected by a third flexible hose to pressure gauge 66. Pressure gauge 66 provides necessary pressure information. Pressure is provided by squeezing bulb 52 until the desired pressure, as indicated by pressure gauge 66, is registered. At that time, valve 56 can be closed to retain pressure. If a jell or liquid is used as a lubricant, hose 60 is preloaded with the desired material. If gas is to be used as a lubricant it is introduced into intake 54 by supplemental equipment, not shown.

Probe 68 is shown in **Figs. 12 and 12A**. Proximal end 70 of probe 68 has a series of ridges 71 extending around the circumference arranged and sized to engage the distal end of distal hose 60 from pressure apparatus 50 for attachment means to provide pressure to the probe. While ridges are shown in this embodiment any means that would provide a similar mechanical connection can be used to achieve the same result. Probe 68 has a distal end, a proximal end and a lumen 72 extending from the proximal end adjacent to but not completely to the distal end. Lumen 72 thus provides a passageway from the proximal end through probe 68 which is blocked on the distal end. Lumen 72 communicates with a number of distal holes 74 near the distal end of probe 68. Distal holes 74 are arranged in sets of three aligned with the longitudinal axis of probe 68. Several sets of these distal holes 74 are placed around the circumference of probe 68. The distal end of probe 68 is rounded for ease of insertion.

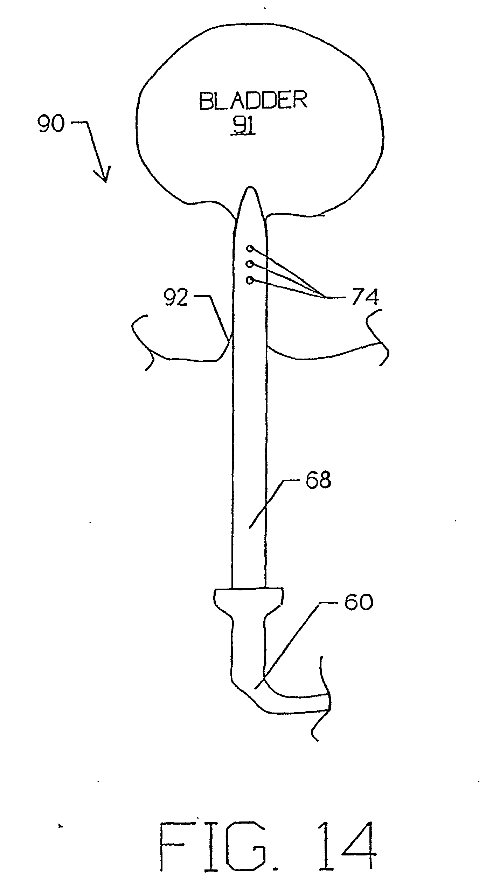

When the distal end of distal hose 60 from pressure apparatus 50 is connected to probe 68 and lubricating material within the apparatus is pressurized, as described earlier, this lubricating material will be forced into the probe through lumen 72 and outwardly through distal holes 74. This will assist in placing probe 68 within the urethra. This process is shown in Fig. 14. That portion of the patient's body 90, which is adjacent to and surrounding bladder 91 and urethra 92 is shown with probe 68 in place within the urethra.

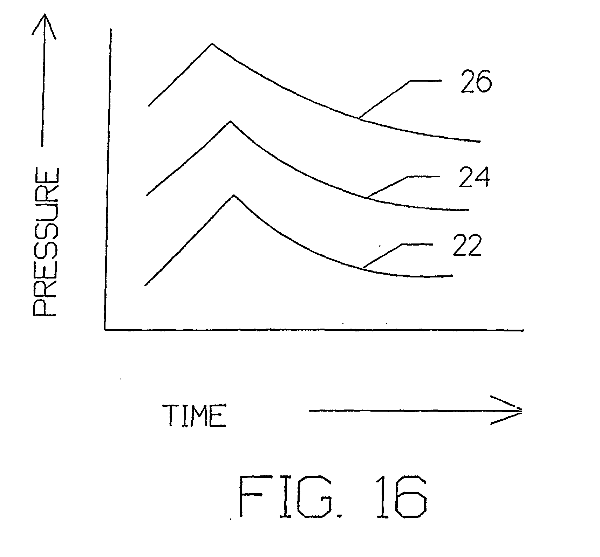

A set of probes 68, ranging in diameter from 22 to 32 millimeters in steps of two millimeters is typical of those used to determine the diameter of the urethra. In Fig. 16 typical pressure curves for a probe having 22, 24 and 26 millimeter diameters, labeled 22, 24 and 26 respectively on the respective curves, are shown. These are typical curves obtained over time as pressure is applied to these probes. Knowledge of desirable pressure ranges permits the physician to select the appropriate diameter to be used for the mount.

Retriever 76 is shown in **Figs. 11 and 11A**. A series of grooves 78 about the proximal end of retriever 76 provides engagement means for the distal end of distal hose 60 from pressure apparatus 50. Retriever 76 has a distal end, a proximal end, a shoulder around the distal end, and a lumen 80 extending from the proximal end through the retriever to the shoulder. Lumen 80 thus provides a passageway from the proximal end through receiver 76 blocked at the distal end. Lumen 80 communicates on the distal end of probe 76 with holes 82 around the circumference of shoulder. Boles 82 are located on shoulder to ensure that lubricating material can pass freely from lumen 80 through holes 82 without being blocked by the walls of urethra 92.

Shoulder 77 has threads 84 about its circumference. Shoulder 73 and threads 84 are sized to fit within and mate with a threaded hole in the base of mount 2, not shown in this figure, to provide mechanical connection means between retriever 76 and mount 2 arranged such that the retriever is aligned with the mount. While a threaded connection is illustrated here, any mechanical connection that would attach the retriever to the mount with the same alignment and force would be acceptable for this use.

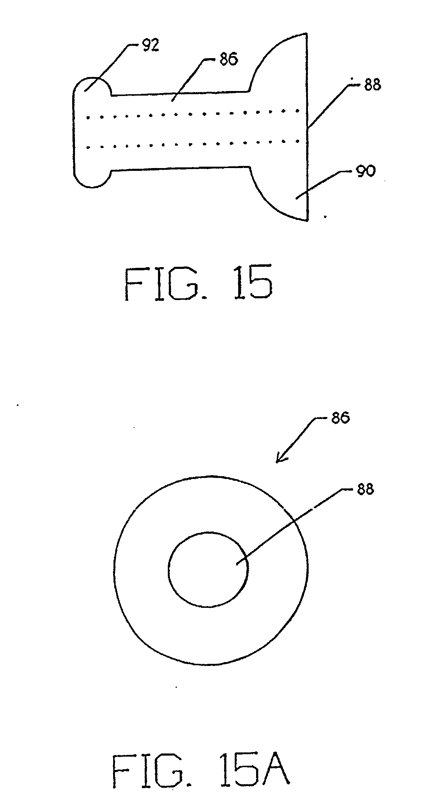

Sleeve 86, shown in **Figs. 15 and 15A**, has hole 88 extending completely through the sleeve from the smaller distal end 90 to the larger proximal end 92. Hole 88 is sized to fit slideably over retriever 76 such that little or no pressurized lubricant can flow between them.

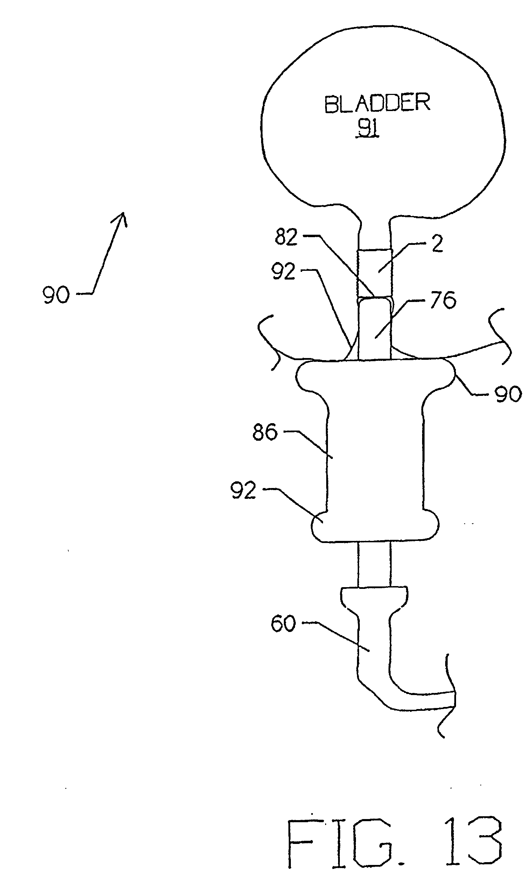

Sleeve 86 is used to contain pressurized lubricant within the urethra when mount 2 is being placed within or removed from urethra 92, as shown in **Fig. 13**. That portion of the patient's body 90, which includes bladder 91 and urethra 92, is shown with mount 2 in place within the urethra. Mount 2 has a threaded proximal hole which mates with thread 84 of retriever 76, described earlier, which holds the retriever securely in place as shown here.

In operation, with the apparatus arranged as shown in **Fig. 13**, with sleeve 86 positioned over probe 76 with the distal end 92 adjacent to the patient's body 90, lubricant can be introduced into urethra 92 through retriever 76. Distal hose 60 extending from pressure apparatus 50, not shown in this figure, provides lubricant through lumen 80 and distal holes 82 of retriever 86 to the volume between the shoulder 77 of the retriever and urethra 92. With sleeve 90 pressed against patient 90 and bulb 52 squeezed, the lubricant forced into urethra 92 will build in pressure and force the walls of the urethra outward. This both expands urethra 92 and simultaneously permits the lubricant to flow between the outer surface of mount 2 and the urethra.

In **Fig. 13**, mount 2 is already in place within urethra 92 and can be removed after lubricant has flown completely around the mount, by pulling retriever 76 outward until the mount is freed. If mount 2 were being placed within urethra 92 the apparatus would be arranged exactly the same as before, excepting that the mount would be forced into urethra 92 using retriever 76, while lubricant from retriever 76 contained by sleeve 86, would assist in lubricating and expanding the urethra ahead of the mount.

Having thus described the preferred embodiments of the present invention, those of skill in the art will readily appreciate the other useful embodiments within the scope of the claims hereto attached.

## Claims

1. Intraurethral bladder control apparatus comprising a valve assembly (20); an input orifice (11) in said valve assembly; an output orifice in said valve assembly; a fluid flow path (8) between the input (11) and output orifices; a moveable valve (17) mounted in the valve assembly and moveable between a closed location adjacent the input orifice (11) and an open position spaced from the input orifice (11); valve closing means for normally holding the valve in the closed position and responsive to a predetermined pressure to allow movement of the valve to the open position; and valve control means mounted in the valve assembly fluid flow path (8) and responsive to the flow of fluid to hold the valve (17) in the open position when the fluid flow rate is above a predetermined rate **characterised in that** it comprises a housing having a textured surface (16) and an inner chamber and the valve control means comprises means for inducing a negative pressure under Bernoulli's principle in the presence of a fluid flow.

2. The apparatus in accordance to claim 1 **characterised in that** said housing is cylindrical.

3. The apparatus in accordance to claim 1 or 2 **characterised in that** the valve closing means comprises a spring (4) and spring tension adjustment means (5).

4. The apparatus of claim 3 **characterised in that** the valve control means comprises:
a. a first valve ring (7) mounted on the moveable valve and within the fluid flow path (8) within the valve assembly;
b. a second valve ring (3) mounted on the connection means and within the fluid flow path (8);
c. a spacer (6) mounted in the valve assembly for maintaining a minimum space between the first and second rings; and
d. the first and second rings (7,3) cooperating in the prsence of said fluid flow of a predetermined level to induce said negative force according to Bernoulli's principle for holding the moveable valve open.

5. The apparatus in accordance to claim 4 **characterised in that** the valve assembly (20) includes adjustment control means (10) for controlling the bias between said srping (4) and said spring tension adjustment means (5) to set the pressures at which the valve assembly will open and close.

6. the apparatus of claim 5 including means for operating the adjustment control means (10) from a point external to the body.

## Patentansprüche

1. Apparat zur Regulierung der intraurethralen Harnblase, mit einem Ventilaufbau (20); einer im Ventilaufbau befindlichen Einlassöffnung (11); einer im Ventilaufbau befindlichen Auslassöffnung; einem Flüssigkeitsströmungsverlauf (8) zwischen der Einlass- (11) und Auslassöffnung; einem in den Ventilaufbau eingebauten beweglichen Ventil (17), das zwischen einer der Einlassöffnung benachbarten (11) geschlossenen Stellung und einer von der Einlassöffnung (11) beabstandeten geöffneten Stellung hin und her beweglich ist; einem Ventilverschlussmittel, mit dem das Ventil normalerweise in der geschlossen Stellung gehalten wird und das auf einen vorgegebenen Druck hin anspricht, um das Ventil in die geöffnete Stellung zu bringen; sowie einem in den Flüssigkeitsströmungsverlauf (8) des Ventilaufbaus eingebauten Ventilregulierungsmittel, das auf die Flüssigkeitsströmung dahingehend anspricht, dass das Ventil (17) in der geöffneten Stellung gehalten wird, wenn die Strömungsgeschwindigkeit der Flüssigkeit über einem vorgegebenen Wert liegt, **dadurch gekennzeichnet, dass** der genannte Apparat ein Gehäuse mit texturierter Oberfläche (16) sowie eine Innenkammer aufweist und das Ventilregulierungsmittel ein Mittel aufweist, das bei Vorliegen einer Flüssigkeitsströmung einen Unterdruck nach dem Bemoulli'schen Prinzip erzeugt.

2. Apparat nach Anspruch 1, **dadurch gekennzeichnet, dass** das Gehäuse zylindrisch ist.

3. Apparat nach Anspruch 1 oder 2, **dadurch gekennzeichnet, dass** das Ventilverschlussmittel eine Feder (4) sowie ein Mittel zur Einstellung der Federspannung (5) aufweist.

4. Apparat nach Anspruch 3, **dadurch gekennzeichnet, dass** das Ventilregulierungsmittel:
a. einen in das bewegliche Ventil und innerhalb des Flüssigkeitsströmungsverlaufs (8) innerhalb des Verntilaufbaus eingebauten ersten Ventilring (7);
b. einen in das Verbindungsmittel und innerhalb des Flüssigkeitsströmungsverlaufs (8) eingebauten zweiten Ventilring (3);
c. ein in den Ventilaufbau eingebautes Abstandstück (6) zur Aufrechterhaltung eines Mindestabstands zwischen dem ersten und zweiten Ring aufweist; wobei
d. der erste und zweite Ring (7, 3) bei Vorliegen einer Flüssigkeitsströmung in der vorgegebenen Höhe so miteinander in Wechselwirkung treten, dass ein Unterdruck nach dem Bernoulli'schen Prinzip erzeugt wird, wodurch das bewegliche Ventil in geöffneter Stellung gehalten wird.

5. Apparat nach Anspruch 4, **dadurch gekennzeichnet, dass** der Ventilaufbau (20) ein Mittel zur Einstellungsregulierung (10) aufweist, mit dem die Vorbelastung zwischen der Feder (4) und dem Mittel zur Einstellung der Federspannung (5) reguliert wird, zwecks Einstellung des Drucks, bei dem der Ventilaufbau öffnet und schließt.

6. Apparat nach Anspruch 5, mit einem Mittel zur Bedienung des Mittels zur Einstellungsregulierung (10) von einer Stelle außerhalb des Körpers.

## Revendications

1. Appareil intra-urétéral destiné à contrôler la vessie, comprenant un système de valve (20), un orifice d'entrée (11) de ce système de valve, un orifice de sortie de ce système de valve, un conduit de circulation du fluide (8) entre l'orifice d'entrée (11) et celui de sortie, une valve mobile (17) montée sur le système de valve et mobile entre une position fermée adjacente de l'orifice d'entrée (11) et une position ouverte écartée de l'orifice d'entrée (11), un dispositif de fermeture de la valve permettant de retenir normalement la valve sur sa position fermée et répondant à une pression prédéterminée afin de permettre le mouvement de la valve vers la position ouverte et un dispositif de contrôle de la valve monté sur le conduit de circulation du fluide du système de valve (8), répondant à la circulation du fluide destiné à maintenir la valve (17) en position ouverte lorsque le débit de circulation du fluide se trouve au-dessus d'un débit prédéterminé, **caractérisé par le fait qu'**il comporte un logement à surface texturée (16), une chambre intérieure et un dispositif de contrôle de la valve comprenant un dispositif destiné à exercer une pression négative conformément au principe de Bernouilli en présence d'un flux de fluide.

2. Appareil correspondant à la revendication 1 **caractérisé par le fait que** ce logement est cylindrique.

3. Appareil correspondant à la revendication 1 ou 2 **caractérisé par le fait que** le dispositif de fermeture de la valve comprend un ressort (4) et un dispositif de réglage de la tension du ressort (5).

4. Appareil correspondant à la revendication 3 **caractérisé par le fait que** le dispositif de contrôle de la valve comprend :
a. une première bague de valve (7) montée sur la valve mobile et dans le conduit de circulation du fluide (8) à l'intérieur du système de valve
b. une deuxième bague de valve (3) montée sur le dispositif de connexion et à l'intérieur du conduit de circulation du fluide
c. un séparateur (6) monté sur le système de valve afin de maintenir un espace minimal entre le premier et le second anneau et
d. la première et la seconde bagues (7,3) qui contribuent en présence de cette circulation de fluide à assurer un niveau prédéterminé permettant d'obtenir cette force négative conformément au principe de Bernouilli afin de maintenir la valve mobile ouverte.

5. Appareil correspondant à la revendication 4 **caractérisé par le fait que** le système de valve (20) comprend un dispositif de contrôle du réglage (10) destiné à contrôler la déviation entre le ressort (4) et le dispositif de réglage de la tension du ressort (5) afin d'établir les pressions auxquelles le système de valve s'ouvrira et se fermera.

6. Appareil correspondant à la revendication 5 comportant un dispositif destiné à faire fonctionner le dispositif de contrôle du réglage (10) d'un point exteme au corps.
